(19) Europäisches Patentamt
European Patent Office
Office européen des brevets

(11) Veröffentlichungsnummer: **0 075 821**
**B1**

# (12) EUROPÄISCHE PATENTSCHRIFT

(45) Veröffentlichungstag der Patentschrift: **02.01.85**

(21) Anmeldenummer: **82108649.3**

(22) Anmeldetag: **18.09.82**

(51) Int. Cl.³: **C 07 C 103/46**, C 07 D 307/68, C 07 C 131/00, A 01 N 37/20, A 01 N 37/36, A 01 N 43/50, A 01 N 43/08, A 01 N 43/10, A 01 N 37/50 // C07C85/24, C07C85/06, C07C101/14, C07D307/32, C07D333/48, C07D261/18, C07D233/56, C07D231/12, C07D249/08

(54) **N-Substituierte Carbonsäureamide, Verfahren zu ihrer Herstellung und diese enthaltende Fungizide.**

(30) Priorität: **28.09.81 DE 3138502**

(43) Veröffentlichungstag der Anmeldung: **06.04.83 Patentblatt 83/14**

(45) Bekanntmachung des Hinweises auf die Patenterteilung: **02.01.85 Patentblatt 85/1**

(84) Benannte Vertragsstaaten: **AT BE CH DE FR GB IT LI NL SE**

(56) Entgegenhaltungen:
**EP - A - 0 067 382**
**FR - A - 1 353 476**

(73) Patentinhaber: **BASF Aktiengesellschaft, Carl-Bosch-Strasse 38, D-6700 Ludwigshafen (DE)**

(72) Erfinder: **Graf, Hermann, Dr., Ginsterstrasse 15, D-6704 Mutterstadt (DE)**
Erfinder: **Goetz, Norbert, Dr., Schoefferstrasse 25, D-6520 Worms 1 (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

**EP 0 075 821 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Carbonsäureamide von 2,6-Dimethylcyclohexyl-aminen, Verfahren zu ihrer Herstellung sowie ihre Verwendung als Fungizide. Sie betrifft außerdem sterisch weitgehend einheitliche N-substituierte Carbonsäureamide von 2,6-Dimethylcyclohexylaminen.

Es ist bekannt, Zink-ethylen-1,2-bis(dithiocarbamat) oder N-Trichlormethylthio-tetrahydrophthalimid als Fungizide in Landwirtschaft und Gartenbau zu verwenden. Die genannten Verbindungen sind gute Mittel zur Bekämpfung von pilzlichen Krankheiten (vgl. z. B. R. Wegler, ›Chemie der Pflanzenschutz- und Schädlingsbekämpfungsmittel‹, Bd. 2, S. 65ff., S. 109, sowie Bd. 4, S. 139 und 191, Springer Verlag, Berlin/Heidelberg/New York, 1970 bzw. 1977). Jedoch sind diese Fungizide nach erfolgter Infektion nicht verwendbar, und ihre Wirkung bei niedrigen Aufwandkonzentrationen genügt den Anforderungen der Praxis nicht.

Es ist ferner bekannt, substituierte N-Alkyl-N-cyclohexyl-chloracetamide als Herbizide zu verwenden (FR-A 13 53 476). Die bekannten Verbindungen haben eine andere chemische Struktur als die neuen Verbindungen, so daß diese durch die bekannten Verbindungen nicht nahegelegt werden.

Es wurde nun gefunden, daß N-substituierte Carbonsäureamide der 2,6-Dimethyl-cyclohexyl-amine der Formel

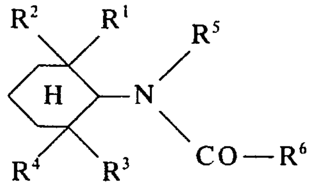

(I)

in der

$R^1$ und $R^3$ Methyl,
$R^2$ und $R^4$ Wasserstoff,
$R^5$ (1-Methoxycarbonyl)-2-ethyl oder alpha-Butyro-gamma-lactonyl und
$R^6$ Chlormethyl, Dichlormethyl, Methoxymethyl, 1-Methoxy-ethyl, Carbonylmethyl, 1-[O-(Methyl)-oximino]-ethyl, 1-[O-(Allyl)-oximino-ethyl, 1-[O-(2,6-Dichlorbenzyl)-oximino]-ethyl, 2-Furyl, 2-Thienyl, 5-Isoxazolyl, 1-Pyrazolylmethyl, 1-Imidazolyl-methyl oder 1-(1,2,4-Triazolyl)-methyl

bedeutet, eine gute fungizide Wirkung aufweisen.

Es wurde ferner gefunden, daß die sterisch weitgehend einheitlichen N-substituierten Carbonsäureamide der Formel

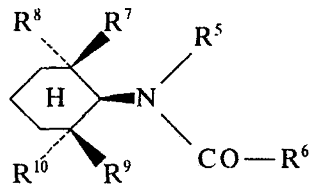

(I)

in der $R^7$ und $R^9$ Methyl, $R^8$ und $R^{10}$ Wasserstoff oder $R^8$ und $R^9$ Wasserstoff, $R^7$ und $R^{10}$ Methyl bedeuten und $R^5$ und $R^6$ die obengenannten Bedeutungen haben, ebenfalls eine gute fungizide Wirkung haben.

Bei den Verbindungen mit den Resten $R^7$ und $R^9$ = Methyl handelt es sich um die cis-cis-Isomeren, die eine besonders gute fungizide Wirkung haben, während die Verbindungen mit den Resten $R^7$ und $R^{10}$ = Methyl die cis-trans-Isomeren und $R^8$ und $R^{10}$ = Methyl die trans-trans-Isomeren sind.

Die neuen N-(2,6-Dimethyl-cyclohexyl)-carbonsäure-amide besitzen im 2-Kohlenstoff des Butyrolacton-Rings bzw. im 2-Kohlenstoff-Atom des Propionsäure-ester-restes ein Asymmetrie-Zentrum, desgleichen im 1-Kohlenstoff des cis,trans-2,6-Dimethyl-cyclohexylamin-Teils. Zudem können durch eingeschränkte Drehbarkeit um die C—N-Bindung im Amin-Teil bei Raumtemperatur stabile Rotamere (Atropisomere) entstehen.

Mit üblichen Methoden können die reinen optischen Enantiomeren bzw. Diastereomeren getrennt werden. Die vorliegende Erfindung umfaßt sowohl die einzelnen Isomeren als auch die entsprechenden Gemische.

Die Herstellung der neuen Verbindungen kann in der Weise vorgenommen werden, daß man ein Cyclohexylamin der Formel

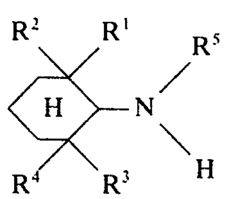

(II)

a) mit einem Säurehalogenid der Formel

$$R^6—CO—X \quad \text{(III)}$$

wobei X Chlor oder Brom bedeutet, oder

b) mit einem Säureanhydrid der Formel

$$R^6—CO—O—CO—R^{11} \quad \text{(IV)}$$

wobei $R^{11}$ die Bedeutung Alkyl oder O-Alkyl hat und $R^6$ die im Anspruch 1 genannten Bedeutungen hat,

gegebenenfalls in einem Lösungs- oder Verdünnungsmittel, gegebenenfalls unter Zusatz einer anorganischen oder organischen Base und gegebenenfalls unter Zusatz eines Reaktionsbeschleunigers bei Temperaturen zwischen 0 und +120°C umsetzt oder

c) mit Chloracetylchlorid umsetzt und das Umsetzungsprodukt mit einem Alkali- oder Erdalkali-Salz der Heterocyclen Pyrazol, Imidazol oder 1,2,4-Triazol in einem Lösungsmittel wie Tetrahydrofuran oder N,N-Dimethylformamid bei Temperaturen zwischen +10 und +100°C umsetzt.

Für die Umsetzungen a) und b) kommen als Lösungsmittel Toluol, Xylol, Dichlormethan, Chloroform oder 1,2-Dichlorethan, als Basen Triethylamin, Natrium- oder Kaliumcarbonat bzw. -hydrogencarbonat, als Reaktionsbeschleuniger Pyridin oder 4-(N,N-Dimethylamino)-pyridin und Temperaturen von +40 bis +80°C in Frage.

Als Lösungsmittel für die Umsetzung c) kommen Tetrahydrofuran oder N,N-Dimethylformamid in Frage.

Die Herstellung der Cyclohexylamine der Formel

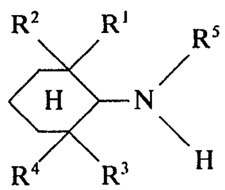

(II)

kann in der Weise erfolgen, daß man ein Cyclohexylamin der Formel

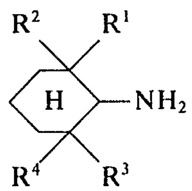

(V)

a) mit Brenztraubensäure-methylester bei Temperaturen von +25 bis +200°C, gegebenenfalls in einem inerten Lösungsmittel, unter Wasserabspaltung zur Verbindung (VI)

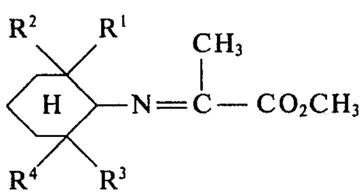

(VI)

umsetzt und diese durch katalytische Hydrierung in das Cyclohexylamin der Formel II mit $R^5$ entsprechend (1-Methoxycarbonyl)-2-ethyl überführt.

Die beiden Stufen können auch in einem Schritt ausgeführt werden, d. h. die Schiffsche Base (VI) kann ohne vorherige Abtrennung direkt reduziert werden.

Als Lösungsmittel seien Ether, insbesondere Tetrahydrofuran, 1,4-Dioxan, 1,2-Dimethoxy-ethan sowie Diethylenglykol-dimethylether genannt, als Katalysator mit Oxiden der Seltenen Erden aktiviertes Palladium auf Kohle oder Aluminiumoxid.

Man kann aber auch das Cyclohexylamin der Formel V

b) mit alpha-Brom-propionsäure-methylester bzw. alpha-Brom-gamma-butyrolacton unter Abspaltung von Bromwasserstoff in einem Lösungs- oder Verdünnungsmittel, gegebenenfalls in Anwesenheit einer anorganischen oder organischen Base und gegebenenfalls in Anwesenheit eines Trockenmittels bei Temperaturen von +40 bis +160°C umsetzen.

Als Lösungsmittel seien genannt: Toluol oder Xylol; als Base: Natrium- oder Kaliumcarbonat bzw. -hydrogencarbonat; als Trockenmittel: Molekularsiebe oder Natrium- bzw. Magnesiumsulfat.

Die Verbindungen der Formel V können hergestellt werden, indem man 2,6-Dimethylphenol mit Ammoniak und Wasserstoff in Anwesenheit eines Katalysators bei Temperaturen zwischen +100 und + 300°C und Drücken von 100 bis 400 bar umsetzt und gegebenenfalls so das erhaltene Gemisch der drei Stereoisomeren einer fraktionierten Destillation unterwirft.

Durch Variation von Druck, Temperatur, Katalysator sowie Reaktionszeit läßt sich das Isomerenverhältnis in weiten Bereichen steuern (DE-OS 30 45 719.3).

Die nachfolgenden Beispiele dienen zur näheren Erläuterung der Erfindung, ohne dieselbe einzuschränken. Wenn nicht anders vermerkt, ist bei Nennung eines Wirkstoffs der Formel (I) stets das racemische Gemisch möglicher optischer Isomerer gemeint. Die Vorschriften betreffen die Herstellung der Zwischenprodukte.

Vorschrift 1

Herstellung von sterisch einheitlichen 2,6-Dimethyl-cyclohexyl-aminen:

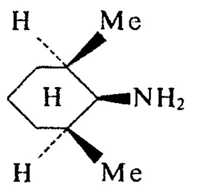

(VII)

cis,cis

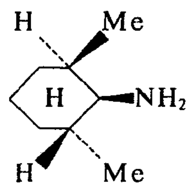

(VIII)

cis,trans

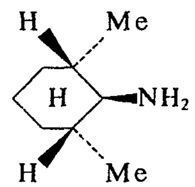

(IX)

trans,trans

$Me = -CH_3$

In einem Rührautoklav mit 10 000 Raumteilen Inhalt werden auf 1650 Gewichtsteile 2,6-Dimethylphenol und 150 Gewichtsteile pulverförmigen Katalysator, bestehend aus Aluminiumoxid mit 10 Gewichtsprozent Palladium und 5,0 Gewichtsprozent Praseodymoxid, 1370 Gewichtsteile Ammoniak aufgepreßt, auf 250°C erhitzt und mit Wasserstoff der Druck auf 300 bar eingestellt. Nach ca. 10 h hat sich ein konstanter Druck ausgebildet. Man läßt abkühlen, filtriert vom Feststoff ab und erhält 1691 Gewichtsteile 2,6-Dimethyl-cyclohexyl-amin, das GC(Gaschromatographie)-Analyse folgende Zusammensetzung aufweist: 14% (VII), 30% (VIII) und 56% (IX). Durch fraktionierte Destillation mit einer Füllkörperkolonne (45 theoretische Böden) erhält man die Isomeren in über 95% Reinheit.

Vorschrift 2

Herstellung von

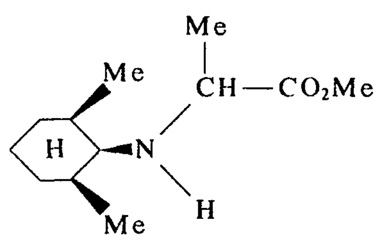

N-[(1-Methoxycarbonyl)-2-ethyl]-cis,cis-2,6-dimethyl-cyclohexyl-amin

(Verbindung Nr. 1a)

102 g (0,803 mol) cis,cis-2,6-Dimethyl-cyclohexyl-amin (VII), 134 g (0,803 mol) alpha-Brom-propionsäuremethylester, 125 g (0,883 mol) wasserfreies Natriumsulfat und 74,2 g (0,883 mol) Natriumhydrogencarbonat werden 10 h bei 100°C unter Stickstoffatmosphäre erhitzt. Nach Abkühlen wird mit 100 ml Toluol verdünnt, abfiltriert, das Filtrat getrocknet und Toluol entfernt. Fraktionierte Destillation führt zu 88,0 g Substanz 1a (99% rein, GC-Analyse) vom Sdp. 56–57°C/0,2 mbar.

Vorschrift 3

Herstellung von

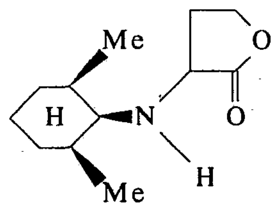

N-(alpha-Butyro-gamma-lactonyl)-cis,cis-2,6-dimethyl-cyclohexyl-amin

(Verbindung Nr. 1b)

107 g (0,842 mol) cis,cis-2,6-Dimethyl-cyclohexylamin (VII), 139 g (0,842 mol) alpha-Brom-butyrolacton, 132 g (0,926 mol) wasserfreies Natriumsulfat und 77,8 g (0,926 mol) Natriumhydrogencarbonat werden 5 h bei 60°C gehalten (Stickstoffatmosphäre). Danach läßt man abkühlen, saugt ab, wäscht mit Toluol nach, schüttelt die organische Phase mit Wasser aus, trocknet sie, engt ein und destilliert über eine Fraktionierungskolonne. Erhalten werden 58 g 99% (GC-Analyse) Flüssigkeit 1b vom Sdp. 95–100°C/0,1 mbar, die beim Stehen erstarrt; Schmp. 48–50°C.

Beispiel 1

Herstellung von

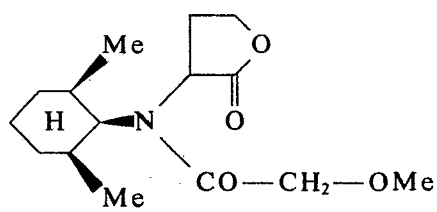

N-(alpha-Butyro-gamma-lactonyl)-N-(cis,cis-2,6-dimethyl-cyclohexyl)-methoxyessigsäure-amid

(Verbindung Nr. 26)

12,6 g (60 mmol) N-substituiertes Amin (1b) erhalten gemäß Vorschrift 3 und 6,5 g (60 mmol) Methoxyacetylchlorid werden in 200 ml trockenem Toluol 3 h bei 60°C in Anwesenheit von 2 g 4-(N,N-Dimethylamino)-pyridin gehalten, wobei ein schwacher Stickstoffstrom durch die Lösung geleitet wird. Nach Abkühlen werden 100 ml Wasser zugesetzt, ausgeschüttelt, die Phasen getrennt, die organische Phase getrocknet und eingeengt. Das zurückbleibende Öl bedarf keiner weiteren Reinigung. $n_D^{22} = 1,4983$.

Nach einer der genannten Methoden können folgende Verbindungen der Formeln (I) und (II) dargestellt werden:

Tabelle 1
N-substituierte Dimethyl-cyclohexyl-amine der Formel (II)
$R^5$ = (1-Methoxycarbonyl)-1-ethyl

| lfd. Nr. | $R^7$ | $R^8$ | $R^9$ | $R^{10}$ | Sdp. bzw. $n_D^{20}$ |
|---|---|---|---|---|---|
| 1a | Me | H | Me | H | 56–57°C/0,2 mbar |
| 2a | Me | H | H | Me | 60–62°C/0,2 mbar; 1,4560 |
| 3a | H | Me | H | Me | 160°C/3,5–4,0 mbar; 1,4565 |

Tabelle 2
N-substituierte Dimethyl-cyclohexyl-amine der Formel (II)
$R^5$ = alpha-Butyro-gamma-lactonyl

| lfd. Nr. | $R^7$ | $R^8$ | $R^9$ | $R^{10}$ | Sdp. bzw. $n_D^{20}$ |
|---|---|---|---|---|---|
| 1b | Me | H | Me | H | 95–100°C/0,1 mbar; Schmp. 48–50°C |
| 2b | Me | H | H | Me | 105–115°C/0,1 mbar |
| 3b | H | Me | H | Me | 160–165°C/0,3–0,5 mbar |

Tabelle 3
N-substituierte Carbonsäureamide der Formel (I)

| lfd. Nr. | $R^7$ | $R^8$ | $R^9$ | $R^{10}$ | $R^5$ | $R^6$ | Schmp./$n_D^{20}$/IR-Banden (fingerprint) |
|---|---|---|---|---|---|---|---|
| 1 | Me | H | Me | H | (1-Methoxy-carbonyl)-1-ethyl | $CH_2OMe$ | Öl; 1440 (br), 1215 (br), 1115 (br), 850, 770 ($cm^{-1}$) |
| 2 | Me | H | H | Me | desgl. | $CH_2OMe$ | Öl; 1440 (br), 1215 (br), 1110 (br), 1010, 932, 912 ($cm^{-1}$) |
| 3 | H | Me | H | Me | desgl. | $CH_2OMe$ | Öl; 1,4785; 1420 (br), 1300 (br), 1210 (br), 1110 (br), 942, 910, 845, 755 ($cm^{-1}$) |
| 7 | Me | H | Me | H | desgl. | $CHCl_2$ | Öl; 1440 (br), 1220 (br), 1200 (br), 810, 780, 665 ($cm^{-1}$) |
| 8 | Me | H | H | Me | desgl. | $CHCl_2$ | |
| 9 | H | Me | H | Me | desgl. | $CHCl_2$ | |
| 10 | Me | H | Me | H | desgl. | 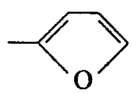 | Öl; |
| 11 | Me | H | H | Me | desgl. | 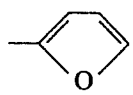 | Öl; 1430 (br), 1375, 130, 1195 (br), 1165, 1310, 1195 (br), 1165 (br), 1005, 845, 750 ($cm^{-1}$) |
| 12 | Me | H | Me | H | desgl. | 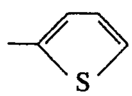 | |
| 13 | Me | H | H | Me | desgl. | 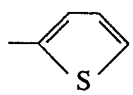 | |
| 14 | Me | H | Me | H | desgl. | 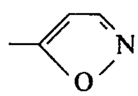 | |
| 15 | Me | H | H | Me | desgl. | 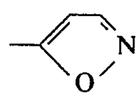 | |
| 16 | Me | H | Me | H | desgl. | 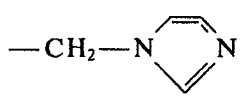 | |
| 17 | Me | H | H | Me | desgl. | 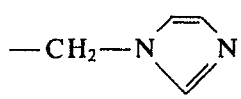 | |
| 18 | Me | H | Me | H | desgl. | 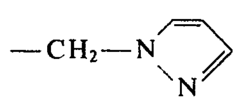 | |

(Fortsetzung)

| lfd. Nr. | $R^7$ | $R^8$ | $R^9$ | $R^{10}$ | $R^5$ | $R^6$ | Schmp./$n_D^{20}$/IR-Banden (fingerprint) |
|---|---|---|---|---|---|---|---|
| 19 | Me | H | H | Me | (1-Methoxy-carbonyl)-1-ethyl | 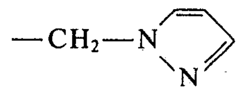 | |
| 20 | Me | H | Me | H | desgl. | 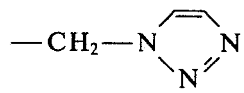 | |
| 21 | Me | H | H | Me | desgl. | 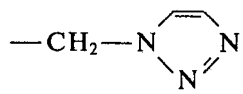 | |
| 22 | H | Me | H | Me | desgl. | COMe | 58–60°C |
| 23 | H | Me | H | Me | desgl. | 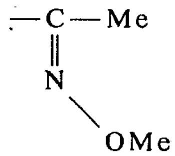 | Öl; 1435 (br), 1220 (br), 1205, 1105, 995 (br), 950, 870, 762, 745, 695 |
| 24 | H | Me | H | Me | desgl. | 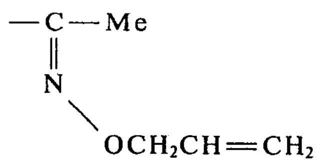 | Öl; 1435 (br), 1220 (br), 1200, 1105, 1025, 991, 925, 865, 762, 745 ($cm^{-1}$) |
| 25 | H | Me | H | Me | desgl. | 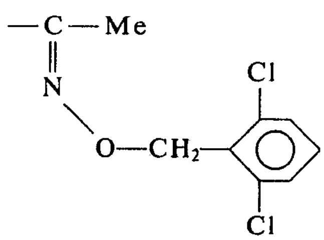 | Öl; 1,5328; 1430 (br), 1365, 1200 (br), 1090, 1030 (br), 990, 910, 875, 770 (br) ($cm^{-1}$) |
| 26 | Me | H | Me | H | alpha-Butyro-gamma-lactonyl | $CH_2OMe$ | Öl; 1,4983; |
| 27 | Me | H | H | Me | desgl. | $CH_2OMe$ | Öl; 1,4936; |
| 28 | H | Me | H | Me | desgl. | $CH_2OMe$ | 80–84°C |
| 29 | Me | H | Me | H | desgl. | $CH_2Cl$ | Öl; |
| 30 | Me | H | H | Me | desgl. | $CH_2Cl$ | Öl; 1,5095 |
| 31 | H | Me | H | Me | desgl. | $CH_2Cl$ | 136–138°C |
| 32 | Me | H | Me | H | desgl. | 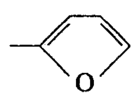 | |
| 33 | Me | H | H | Me | desgl. | 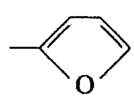 | 127–132°C |
| 34 | Me | H | Me | H | desgl. | 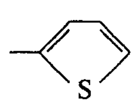 | |

(Fortsetzung)

| lfd. Nr. | $R^7$ | $R^8$ | $R^9$ | $R^{10}$ | $R^5$ | $R^6$ | Schmp./$n_D^{20}$/IR-Banden (fingerprint) |
|---|---|---|---|---|---|---|---|
| 35 | Me | H | H | Me | alpha-Butyro-gamma-lactonyl | 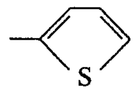 | |
| 36 | Me | H | Me | H | desgl. | 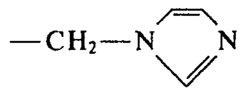 | |
| 37 | Me | H | H | Me | desgl. | 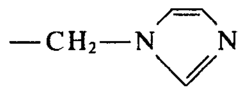 | |
| 38 | Me | H | Me | H | desgl. | 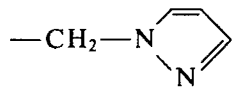 | |
| 39 | Me | H | H | Me | desgl. | 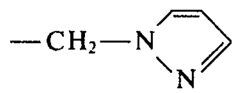 | |
| 40 | Me | H | Me | H | desgl. | 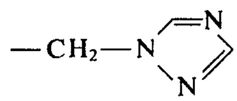 | |
| 41 | Me | H | H | Me | desgl. | 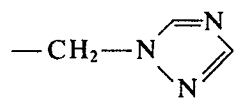 | |
| 42 | H | Me | H | Me | desgl. | CH(Me)OMe | 113–115°C |
| 43 | H | Me | H | Me | desgl. | COMe | 121–124°C |
| 44 | H | Me | H | Me | (1-Methoxy-carbonyl)-1-ethyl | 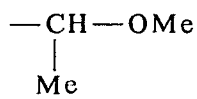 | 79–82°C |
| 45 | H | Me | H | Me | alpha-Butyro-gamma-lactonyl | 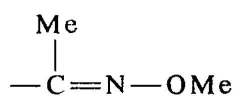 | 60–63°C |
| 46 | H | Me | H | Me | desgl. | 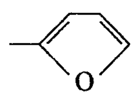 | 133–135°C |
| 47 | H | Me | H | Me | desgl. | 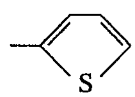 | 126–128°C |
| 48 | H | Me | H | Me | desgl. | 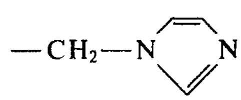 | 76°C (Zers.) |

br = breit

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen,

Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Erysiphe graminis an Getreide, Uncinula necator an Reben, Podophaera leucotricha an Äpfeln, Sphaerotheca fuliginea an Rosen, Erysiphe cichoracearum an Gurken. Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 und 5 kg Wirkstoff je ha. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Darüber hinaus sind viele der neuen Verbindungen systemisch wirksam, so daß über die Wurzelbehandlung auch ein Schutz oberirdischer Pflanzenteile möglich ist.

Die neuen Wirkstoffe können auch zusammen mit anderen Wirkstoffen, z. B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Manganethylenbisdithiocarbamat,
Mangan-Zinkethylenbisdithiocarbamat,
Ammoniak-Komplex von Zink-(N,N-ethylen-bis-dithiocarbamat),
N-Trichlormethylthio-tetrahydrophthalimid,
N-Trichlormethyl-phthalimid,
5-Ethoxy-3-trichlormethyl-1,2,3-thiadiazol,
2-Methoxycarbonylamino-benzimidazol,
2-Rhodanmethylthiobenzthiazol,
1,4-Dichlor-2,5-dimethoxybenzol,
2,3-Dichlor-6-methyl-1,4-oxathiin-5-carbonsäureanilid,
2-Methyl-5,6-dihydro-4-H-pyran-3-carbonsäure-anilid,
2,4,5-Trimethyl-furan-3-carbonsäureanilid,
2-Methyl-furan-3-carbonsäureanilid,
2,5-Dimethyl-furan-3-carbonsäurecyclohexylamid,
N-Cyclohexyl-N-methoxy-2,5-dimethyl-furan-3-carbonsäure-amid,
5-Methyl-5-vinyl-3-(3,5-dichlorphenyl)-2,4-dioxo-1,3-oxazolidin,
3-(3,5-Dichlorphenyl)-5-methyl-5-methoxymethyl-1,3-oxazolidin-2,4-dion.

Die folgende Liste von Fungiziden, mit denen die neuen Verbindungen kombiniert werden können, soll die Kombinationsmöglichkeiten erläutern, nicht aber einschränken.

Fungizide, die mit den erfindungsgemäßen Wirkstoffen kombiniert werden können, sind beispielsweise:

Dithiocarbanate und deren Derivate, wie
Ferridimethyldithiocarbamat,
Zinkdimethyldithiocarbamat,
Zinkethylenbisdithiocarbamat,
Tetramethylthiuramdisulfide,
Zink-(N,N-propylen-bis-dithiocarbamat),
Ammoniak-Komplex von Zink-(N,N'-propylen-bis-dithiocarbamat) und
N,N'-Polypropylen-bis-(thiocarbamoyl)-disulfid.

Nitroderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat.

Heterocyclische Strukturen, wie
2-Heptadecyl-2-imidazolin-acetat,
2,4-Dichlor-6-(o-chloranilino)-s-triazin,
O,O-Diethyl-phthalimidophonothioat,
5-Amino-1-(bis-(dimethylamino)-phosphinyl)-3-phenyl-1,2,4-triazol),
2,3-Dicyano-1,4-dithiaanthrachinon,
2-Thio-1,3-dithio-(4,5-b)-chinoxalin,
1-(Butylcarbamoyl)-2-benzimidazol-carbaminsäuremethyl-ester,
4-(2-Chlorphenylhydrazono)-3-methyl-5-isoxazolon,
Pyridin-2-thio-1-oxid,

8-Hydroxychinolin bzw. dessen Kupfersalz,
2,3-Dihydro-5-carboxanilido-6-methyl-1,4-oxathiin-4,4-dioxid,
2,3-Dohydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid,
2-(Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2-Methyl-benzoesäure-anilid,
2-Jod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
$\alpha$-(2-Chlorphenyl)-$\alpha$-(4-chlorphenyl)-5-pyrimidin-methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Gießen angewendet. Die Aufwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Für die folgenden Versuche wurden als bekannte Vergleichswirkstoffe die folgenden Verbindungen verwendet:

N-Trichlormethylthio-tetrahydrophthalimid (Verbindung A),
Zink-ethylen-1,2-bis-dithiocarbamat (Verbindung B).

Versuch 1

Wirksamkeit gegen Phytophthora infestans an Tomaten

Blätter von Topfpflanzen der Sorte ›Große Fleischtomate‹ werden mit wäßriger Spritzbrühe, die 80% Wirkstoff und 20%Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise die Verbindungen 1 und 5 bei Anwendung als 0,025%ige Spritzbrühe eine bessere fungizide Wirkung (beispielsweise 90%) zeigen als die bekannten Wirkstoffe A und B (beispielsweise 70%).

Beispiele für Zubereitungen sind:

I. Man vermischt 90 Gew.-Teile der Verbindung 1 mit 10 Gew.-Teilen N-Methyl-alpha-pyrrolidon und erhält eine Lösung, die zur Anwendung in Form kleinster Tropfen geeignet ist.

II. 20 Gew.-Teile der Verbindung 5 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung 1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung 5 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung 1 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung 5 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung 5 werden mit 10 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 1 werden mit 2 Teilen Calciumsalz der Dodecylbenzolsulfonsäure, 8 Teilen Fettalkohol-polyglykolether, 2 Teilen Natriumsalz eines Phenolsulfonsäure-Harnstoff-Formaldehyd-Kondensats und 68 Teilen eines paraffinischen Mineralöls innig vermischt. Man erhält eine stabile ölige Dispersion.

**Patentansprüche für die Vertragsstaaten: BE, CH, DE, FR, GB, IT, LI, NL, SE**

1. N-substituiertes Carbonsäureamid der Formel

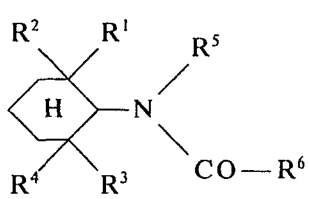

(I)

in der
$R^1$ und $R^3$ Methyl,
$R^2$ und $R^4$ Wasserstoff,
$R^5$ (1-Methoxycarbonyl)-1-ethyl oder alpha-Butyro-gamma-lactonyl und
$R^6$ Chlormethyl, Dichlormethyl, Methoxymethyl, 1-Methoxy-ethyl, Carbonylmethyl, 1-[O-(Methyl)-oximino]-ethyl, 1-[O-(Allyl)-oximino]-ethyl, 1-[O-(2,6-Dichlorbenzyl)-oximino]-ethyl, 2-Furyl, 2-Thienyl, 5-Isoxazolyl, 1-Pyrazolylmethyl, 1-Imidazolyl-methyl oder 1-(1,2,4-Triazolyl)-methyl
bedeutet außer der Verbindung N-(1-Methoxycarbonyl-1-ethyl)-N-(2,6-dimethylcyclohexyl)-chloressigsäureamid.

2. Sterisch weitgehend einheitliches N-substituiertes Carbonsäureamid gemäß Anspruch 1 mit der Formel

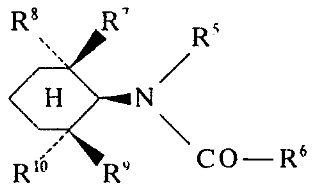

(I)

in der $R^7$ und $R^9$ Methyl, $R^8$ und $R^{10}$ Wasserstoff oder $R^8$ und $R^9$ Wasserstoff, $R^7$ und $R^{10}$ Methyl bedeuten und $R^5$ und $R^6$ die im Anspruch 1 angegebenen Bedeutungen haben.

3. Verbindung gemäß Anspruch 1, dadurch gekennzeichnet, daß $R^5$ 1-Methoxycarbonyl-1-ethyl und $R^6$ Methoxymethyl bedeutet.

4. Fungizid, enthaltend ein N-substituiertes Carbonsäureamid der Formel

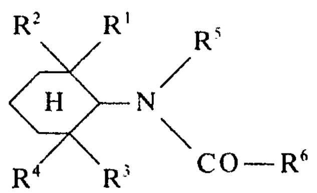

(I)

in der
$R^1$ und $R^3$ Methyl,
$R^2$ und $R^4$ Wasserstoff,
$R^5$ (1-Methoxycarbonyl)-1-ethyl oder alpha-Butyro-gamma-lactonyl und
$R^6$ Chlormethyl, Dichlormethyl, Methoxymethyl, 1-Methoxy-ethyl, Carbonylmethyl, 1-[O-(Methyl)-oximino]-ethyl, 1-[O-(Allyl)-oximino]-ethyl, 1-[O-(2,6-Dichlorbenzyl)-oximino]-ethyl, 2-Furyl, 2-Thienyl, 5-Isoxazolyl, 1-Pyrazolylmethyl, 1-Imidazolyl-methyl oder 1-(1,2,4-Triazolyl)-methyl bedeutet.

5. Fungizid, enthaltend ein sterisch weitgehend einheitliches N-substituiertes Carbonsäureamid gemäß Anspruch 1 mit der Formel

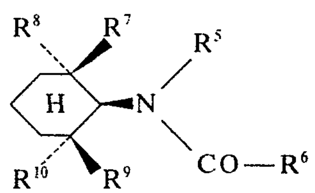

(I)

in der $R^7$ und $R^9$ Methyl, $R^8$ und $R^{10}$ Wasserstoff oder $R^8$ und $R^9$ Wasserstoff, $R^7$ und $R^{10}$ Methyl bedeuten und $R^5$ und $R^6$ die im Anspruch 1 angegebenen Bedeutungen haben.

6. Fungizid, enthaltend eine Verbindung gemäß Anspruch 4, dadurch gekennzeichnet, daß $R^5$ 1-Methoxycarbonyl-1-ethyl und $R^6$ Chlormethyl oder Methoxymethyl bedeutet.

7. Verfahren zur Bekämpfung von Pilzen, dadurch gekennzeichnet, daß man eine fungizid wirksame Menge einer Verbindung gemäß Anspruch 4 auf die Pilze oder die vor Pilzbefall zu schützenden Pflanzen, Flächen oder Saatgüter einwirken läßt.

8. Verfahren zur Herstellung von Verbindungen gemäß Anspruch 1, dadurch gekennzeichnet, daß man Cyclohexylamin der Formel

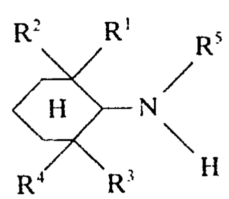

(II)

a) mit einem Säurehalogenid der Formel

$$R^6—CO—X \qquad (III)$$

wobei X Chlor oder Brom bedeutet, oder

b) mit einem Säureanhydrid der Formel

$$R^6—CO—O—CO—R^{11} \qquad (IV)$$

wobei $R^{11}$ die Bedeutung Alkyl oder O-Alkyl hat und $R^6$ die im Anspruch 1 genannten Bedeutungen hat,

gegebenenfalls in einem Lösungs- oder Verdünnungsmittel, gegebenenfalls unter Zusatz einer anorganischen oder organischen Base und gegebenenfalls unter Zusatz eines Reaktionsbeschleunigers bei Temperaturen zwischen 0 und +120°C umsetzt oder

c) mit Chloracetylchlorid umsetzt und das Umsetzungsprodukt mit einem Alkali- oder Erdalkali-Salz der Heterocyclen Pyrazol, Imidazol oder 1,2,4-Triazol in einem Lösungsmittel bei Temperaturen zwischen +10 und +100°C umsetzt.

**Patentansprüche für den Vertragsstaat: AT**

1. Fungizid, enthaltend ein N-substituiertes Carbonsäureamid der Formel

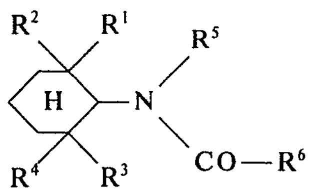

(I)

in der

$R^1$ und $R^3$ Methyl,
$R^2$ und $R^4$ Wasserstoff,
$R^5$ (1-Methoxycarbonyl)-1-ethyl oder alpha-Butyro-gamma-lactonyl und
$R^6$ Chlormethyl, Dichlormethyl, Methoxymethyl, 1-Methoxy-ethyl, Carbonylmethyl, 1-[O-(Methyl)-oximino]-ethyl, 1-[O-(Allyl)-oximino]-ethyl, 1-[O-(2,6-Dichlorbenzyl)-oximino]-ethyl, 2-Furyl, 2-Thienyl, 5-Isoxazolyl, 1-Pyrazolylmethyl, 1-Imidazolyl-methyl oder 1-(1,2,4-Triazolyl)-methyl

bedeutet.

2. Fungizid, enthaltend ein sterisch weitgehend einheitliches N-substituiertes Carbonsäureamid gemäß Anspruch 1 mit der Formel

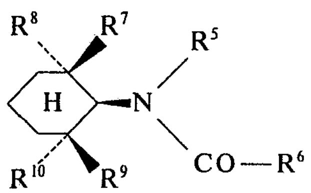

(I)

in der $R^7$ und $R^9$ Methyl, $R^8$ und $R^{10}$ Wasserstoff oder $R^8$ und $R^9$ Wasserstoff, $R^7$ und $R^{10}$ Methyl bedeuten und $R^5$ und $R^6$ die im Anspruch 1 angegebenen Bedeutungen haben.

3. Fungizid, enthaltend eine Verbindung gemäß Anspruch 1, dadurch gekennzeichnet, daß $R^5$ 1-Methoxycarbonyl-1-ethyl und $R^6$ Chlormethyl oder Methoxymethyl bedeutet.

4. Verfahren zur Bekämpfung von Pilzen, dadurch gekennzeichnet, daß man eine fungizid wirksame Menge einer Verbindung gemäß Anspruch 1 auf die Pilze oder die vor Pilzbefall zu schützenden Pflanzen, Flächen oder Saatgüter einwirken läßt.

5. Verfahren zur Herstellung von Verbindungen gemäß Anspruch 1, dadurch gekennzeichnet, daß man Cyclohexylamin der Formel

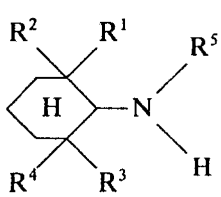

(II)

a) mit einem Säurehalogenid der Formel

$$R^6—CO—X \qquad (III)$$

wobei X Chlor oder Brom bedeutet, oder

b) mit einem Säureanhydrid der Formel

$$R^6—CO—O—CO—R^{11} \qquad (IV)$$

wobei $R^{11}$ die Bedeutung Alkyl oder O-Alkyl hat und $R^6$ die im Anspruch 1 genannten Bedeutungen hat,

gegebenenfalls in einem Lösungs- oder Verdünnungsmittel, gegebenenfalls unter Zusatz einer anorganischen oder organischen Base und gegebenenfalls unter Zusatz eines Reaktionsbeschleunigers bei Temperaturen zwischen 0 und +120°C umsetzt oder

c) mit Chloracetylchlorid umsetzt und das Umsetzungsprodukt mit einem Alkali- oder Erdalkali-Salz der Heterocyclen Pyrazol, Imidazol oder 1,2,4-Triazol in einem Lösungsmittel bei Temperaturen zwischen +10 und +100°C umsetzt.

**Claims for the Contracting States: BE, CH, DE, FR, GB, IT, LI, NL, SE**

1. An N-substituted carboxylic acid amide of the formula

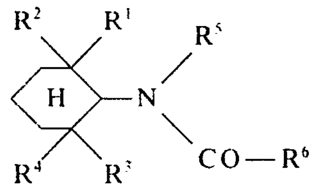

(I)

where

$R^1$ and $R^3$ are each methyl,

$R^2$ and $R^4$ are each hydrogen,

$R^5$ is (1-methoxycarbonyl)-1-ethyl or $\alpha$-butyro-$\gamma$-lactonyl, and

$R^6$ is chloromethyl, dichloromethyl, methoxymethyl, 1-methoxyethyl, carbonylmethyl, 1-[O-(methyl)-oximino]-ethyl, 1-[O-(allyl)-oximino]-ethyl, 1-[O-(2,6-dichlorobenzyl)-oximino]-ethyl, fur-2-yl, thien-2-yl, isoxazol-5-yl, pyrazol-1-ylmethyl, imidazol-1-yl-methyl or 1-(1,2,4-triazolyl)-methyl, except the compound N-(1-methoxycarbonyl-1-ethyl)-N-(2,6-dimethylcyclohexyl)-chloroacetic acid amide.

2. A sterically substantially uniform N-substituted carboxylic acid amide, as claimed in claim 1, of the formula

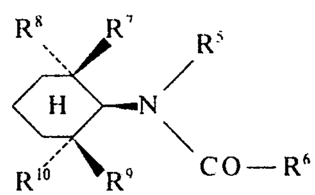

(I)

where $R^7$ and $R^9$ are each methyl and $R^8$ and $R^{10}$ are each hydrogen, or $R^8$ and $R^9$ are each hydrogen and $R^7$ and $R^{10}$ are each methyl, and $R^5$ and $R^6$ have the meanings given in claim 1.

3. A compound as claimed in claim 1, where $R^5$ is 1-methoxycarbonyl-1-ethyl and $R^6$ is methoxymethyl.

4. A fungicide containing an N-substituted carboxylic acid amide of the formula

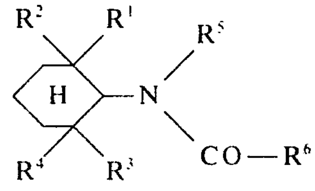

(I)

where

$R^1$ and $R^3$ are each methyl,

$R^2$ and $R^4$ are each hydrogen,

$R^5$ is (1-methoxycarbonyl)-1-ethyl or $\alpha$-butyro-$\gamma$-lactonyl, and

$R^6$ is chloromethyl, dichloromethyl, methoxymethyl, 1-methoxyethyl, carbonylmethyl, 1-[O-(methyl)-oximino]-ethyl, 1-[O-(allyl)-oximino]-ethyl, 1-[O-(2,6-dichlorobenzyl)-oximino]-ethyl, fur-2-yl, thien-2-yl, isoxazol-5-yl, pyrazol-1-ylmethyl, imidazol-1-yl-methyl or 1-(1,2,4-triazolyl)-methyl.

5. A fungicide containing a sterically substantially uniform N-substituted carboxylic acid amide, as claimed in claim 1, of the formula

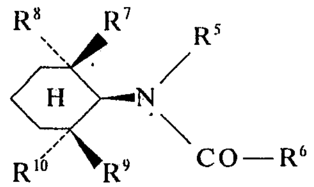

(I)

where $R^7$ and $R^9$ are each methyl and $R^8$ and $R^{10}$ are each hydrogen, or $R^8$ and $R^9$ are each hydrogen and $R^7$ and $R^{10}$ are each methyl, and $R^5$ and $R^6$ have the meanings given in claim 1.

6. A fungicide containing a compound as defined in claim 4, where $R^5$ is 1-methoxycarbonyl-1-ethyl and $R^6$ is chloromethyl or methoxymethyl.

7. A process for combating fungi, wherein a fungicidally effective amount of a compound as defined in claim 4 is allowed to act on the fungi or on the plants, areas or seed to be protected against fungal attack.

8. A process for preparing a compound as claimed in claim 1, wherein a cyclohexylamine of the formula

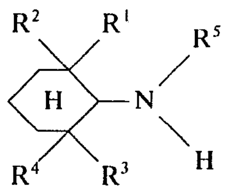

(II)

is reacted

(a) with an acid halide of the formula

$$R^6—CO—X \qquad (III)$$

where X is chlorine or bromine, or

(b) with an acid anhydride of the formula

$$R^6—CO—O—CO—R^{11} \qquad (IV)$$

where $R^{11}$ is alkyl or O-alkyl and $R^6$ has the meanings given in claim 1,

in the presence or absence of a solvent or dilnent, with or without the addition of an inorganic or organic base and with or without the addition of a reaction accelerator, at from 0 to +120°C, or

(c) is reacted with chloroacetyl chloride and the reaction product is reacted with an alkali metal or alkaline earth metal salt of the heterocycles pyrazole, imidazole or 1,2,4-triazole in a solvent at from +10 to +100°C.

**Claims for the Contracting State: AT**

1. A fungicide containing an N-substituted carboxylic acid amide of the formula

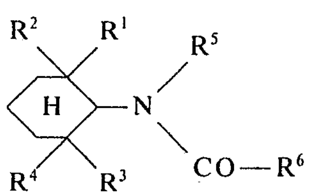

(I)

where

$R^1$ and $R^3$ are each methyl,

$R^2$ and $R^4$ are each hydrogen,

$R^5$ is (1-methoxycarbonyl)-1-ethyl or $\alpha$-butyro-$\gamma$-lactonyl, and

$R^6$ is chloromethyl, dichloromethyl, methoxymethyl, 1-methoxyethyl, carbonylmethyl, 1-[O-(methyl)-oximino]-ethyl, 1-[O-(allyl)-oximino]-ethyl, 1-[O-(2,6-dichlorobenzyl)-oximino]-ethyl, fur-2-yl, thien-2-yl, isoxazol-5-yl, pyrazol-1-ylmethyl, imidazol-1-yl-methyl or 1-(1,2,4-triazolyl)-methyl.

2. A fungicide containing a sterically substantially uniform N-substituted carboxylic acid amide, as claimed in claim 1, of the formula

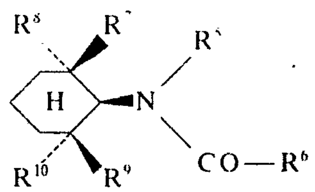

(I)

where $R^7$ and $R^9$ are each methyl and $R^8$ and $R^{10}$ are each hydrogen, or $R^8$ and $R^9$ are each hydrogen and $R^7$ and $R^{10}$ are each methyl, and $R^5$ and $R^6$ have the meanings given in claim 1.

3. A fungicide containing a compound as defined in claim 1, where $R^5$ is 1-methoxycarbonyl-1-ethyl and $R^6$ is chloromethyl or methoxymethyl.

4. A process for combating fungi, wherein a fungicidally effective amount of a compound as defined in claim 1 is allowed to act on the fungi or on the plants, areas or seed to be protected against fungal attack.

5. A process for preparing a compound as defined in claim 1, wherein a cyclohexylamine of the formula

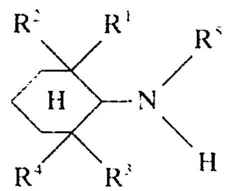

(II)

is reacted

(a) with an acid halide of the formula

$R^6—CO—X$ (III)

where X is chlorine or bromine, or

(b) with an acid anhydride of the formula

$R^6—CO—O—CO—R^{11}$ (IV)

where $R^{11}$ is alkyl or O-alkyl and $R^6$ has the meanings given in claim 1,

in the presence or absence of a solvent or diluent, with or without the addition of an inorganic or organic base and with or without the addition of a reaction accelerator, at from 0 to +120°C, or

(c) is reacted with chloroacetyl chloride and the reaction product is reacted with an alkali metal or alkaline earth metal salt of the heterocycles pyrazole, imidazole or 1,2,4-triazole in a solvent at from +10 to +100°C.

**Revendications pour les Etats contractants: BE, CH, DE, FR, GB, IT, LI, NL, SE**

1. Amine d'acide carboxylique N-substitué de la formule

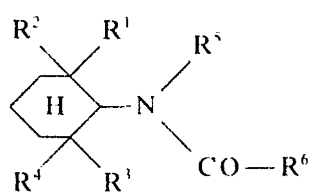

(I)

dans laquelle

$R^1$ et $R^3$ désignent chacun un radical méthyle,

$R^2$ et $R^4$ représentent des atomes d'hydrogène,

$R^5$ est un groupe (1-méthoxy-carbonyl)-1-éthyle ou $\alpha$-butyro-$\gamma$-lactonyle et

$R^6$ est un groupe chloro-méthyle, dichloro-méthyle, méthoxy-méthyle, 1-méthoxy-éthyle, carbonyl-méthyle, 1-[O-(méthyl)-oximino]-éthyle, 1-[O-(allyl)-oximino]-éthyle, 2-furyle, 2-thiényle, 5-isoxazolyle, 1-pyrazolyl-méthyle, 1-imidazolyl-méthyle ou 1-(1,2,4-triazolyl)-méthyle,

excepté le composé amide de l'acide N-(1-méthoxy-carbonyl-1-éthyl)-N-(2,6-diméthyl-cyclohexyl)-chlor-acétique.

2. Amide d'acide carboxylique N-substitué largement homogène du point de vue stérique, selon la revendication 1, de la formule

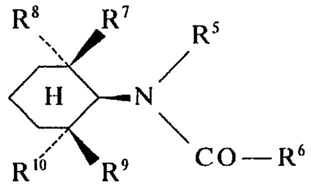

(I)

dans laquelle $R^5$ et $R^6$ possèdent les significations définies dans la revendication 1, $R^7$ et $R^9$ ou $R^7$ et $R^{10}$ désignent des radicaux méthyle et $R^8$ et $R^{10}$ ou $R^8$ et $R^9$ des atomes d'hydrogène.

3. Composé selon la revendication 1, caractérisé en ce que $R^5$ représente un groupe 1-méthoxy-carbonyl-1-éthyle et $R^6$ un groupe méthoxy-méthyle.

4. Composition fongicide, contenant un amide d'acide carboxylique N-substitué de la formule

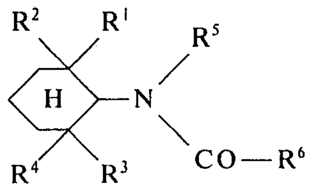

(I)

dans laquelle

$R^1$ et $R^3$ désignent chacun un radical méthyle,

$R^2$ et $R^4$ représentent des atomes d'hydrogène,

$R^5$ est un groupe (1-méthoxy-carbonyl)-1-éthyle ou $\alpha$-butyro-$\gamma$-lactonyle et

$R^6$ est un groupe chloro-méthyle, dichloro-méthyle, méthoxy-méthyle, 1-méthoxy-éthyle, carbonyl-méthyle, 1-[O-(méthyl)-oximino]-éthyle, 1-[O-(allyl)-oximino]-éthyle, 2-furyle, 2-thiényle, 5-isoxazolyle, 1-pyrazolyl-méthyle, 1-imidazolyl-méthyle ou 1-(1,2,4-triazolyl)-méthyle.

5. Composition fongicide, contenant un amide d'acide carboxylique N-substitué largement homogène du point de vue stérique selon la revendication 1, de la formule

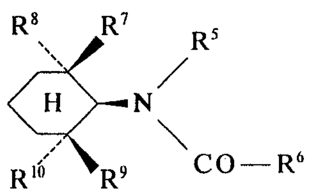

(I)

dans laquelle $R^5$ et $R^6$ possèdent les significations définies dans la revendication 1, $R^7$ et $R^9$ ou $R^7$ et $R^{10}$ désignent des radicaux méthyle et $R^8$ et $R^{10}$ ou $R^8$ et $R^9$ des atomes d'hydrogène.

6. Composition fongicide, contenant un composé selon la revendication 4, caractérisée en ce que $R^5$ représente un groupe 1-méthoxy-carbonyl-1-éthyle et $R^6$ un groupe chlorométhyle ou méthoxy-méthyle.

7. Procédé de lutte contre les champignons, caractérisé en ce que l'on fait agir une quantité efficace du point de vue fongicide d'un composé selon la revendication 4 sur les champignons ou sur les plantes, surfaces ou semences à protéger des champignons.

8. Procédé de préparation de composés selon la revendication 1, caractérisé en ce que l'on fait réagir une cyclohexyl-amine de la formule

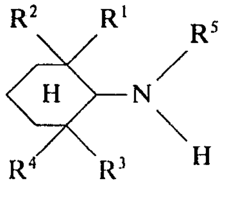

(II)

à des températures comprises entre 0 et +120°C, éventuellement en présence d'un solvant ou diluant et(ou) d'une base inorganique ou organique et(ou) d'un accélérateur de la réaction soit

a) avec un halogénure d'acide de la formule

$$R^6—CO—X \qquad (III)$$

où X = chlore ou brome, soit

b) avec un anhydride d'acide de la formule

$$R^6—CO—O—CO—R^{11} \qquad (IV)$$

où $R^{11}$ désigne un radical alkyle ou O-alkyle et $R^6$ possède les significations définies dans la revendication 1, ou

c) avec le chlorure de chlor-acétyle, puis on fait réagir le produit réactionnel obtenu dans un solvant, à des températures comprises entre +10 et +100°C, avec un sel de métal alcalin ou alcalinoterreux d'un des composés hétérocycliques pyrazole, imidazole ou 1,2,4-triazole.

**Revendications pour l'Etat contractant: AT**

1. Composition fongicide, contenant un amide d'acide carboxylique N-substitué de la formule

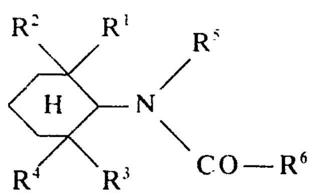

(I)

dans laquelle
$R^1$ et $R^3$ désignent chacun un radical méthyle,
$R^2$ et $R^4$ représentent des atomes d'hydrogène,
$R^5$ est un groupe (1-méthoxy-carbonyl)-1-éthyle ou $\alpha$-butyro-$\gamma$-lactonyle et
$R^6$ est un groupe chloro-méthyle, dichloro-méthyle, méthoxy-méthyle, 1-méthoxy-éthyle, carbonyl-méthyle, 1-[O-(méthyl)-oximino]-éthyle, 1-[O-(allyl)-oximino]-éthyle, 2-furyle, 2-thiényle, 5-isoxazolyle, 1-pyrazolyl-méthyle, 1-imidazolyl-méthyle ou 1-(1,2,4-triazolyl)-méthyle.

2. Composition fongicide, contenant un amide d'acide carboxylique N-substitué largement homogène du point de vue stérique, selon la revendication 1, de la formule

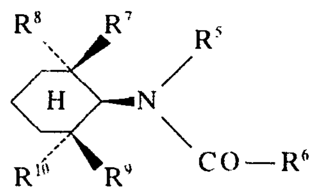

(I)

dans laquelle $R^5$ et $R^6$ possèdent les significations définies dans la revendication 1, $R^7$ et $R^9$ ou $R^7$ et $R^{10}$ désignent des radicaux méthyle et $R^8$ et $R^{10}$ ou $R^8$ et $R^9$ des atomes d'hydrogène.

3. Composition fongicide, contenant un composé selon la revendication 1, caractérisé en ce que $R^5$ représente un groupe 1-méthoxy-carbonyl-1-éthyle et $R^6$ un groupe chlorméthyle ou méthoxyméthyle.

4. Procédé de lutte contre les champignons, caractérisé en ce que l'on fait agir une quantité efficace du point de vue fongicide d'un composé selon la revendication 1 sur les champignons ou sur les plantes, surfaces ou semences à protéger des champignons.

5. Procédé de préparation de composés selon la revendication 1, caractérisé en ce que l'on fait réagir une cyclohexyl-amine de la formule

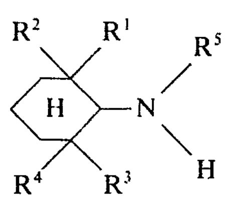

(II)

à des températures comprises entre 0 et +120°C, éventuellement en présence d'un solvant ou diluant et(ou) d'une base inorganique ou organique et(ou) d'un accélérateur de la réaction soit

a) avec un halogénure d'acide de la formule

$$R^6—CO—X \quad (III)$$

où X = chlore ou brome, soit

b) avec un anhydride d'acide de la formule

$$R^6—CO—O—CO—R^{11} \quad (IV)$$

où $R^{11}$ désigne un radical alkyle ou O-alkyle et $R^6$ possède les significations définies dans la revendication 1, ou

c) avec le chlorure de chlor-acétyle, puis on fait réagir le produit réactionnel obtenu dans un solvant, à des températures comprises entre +10 et +100°C, avec un sel de métal alcalin ou alcalinoterreux d'un des composés hétérocycliques pyrazole, imidazole ou 1,2,4-triazole.